# FASCICULE DE BREVET EUROPEEN

(11) **EP 3 664 618 B1**
(45) Date de publication et mention de la délivrance du brevet: **11.11.2026**
(21) Numéro de dépôt: 18765951.1
(22) Date de dépôt: 09.08.2018
(51) Int. Cl.: A01N 63/00

(54) **UTILISATION THERAPEUTIQUE OU NON-THERAPEUTIQUE DE PROTOZOAIRES DU GENRE WILLAERTIA COMME FONGISTATIQUE ET/OU FONGICIDE**
THERAPEUTISCHE ODER NICHTTHERAPEUTISCHE VERWENDUNG VON PROTOZOEN DER WILLAERTIA-GATTUNG ALS FUNGISTATISCHES UND/ODER FUNGIZIDES MITTEL
THERAPEUTIC OR NON-THERAPEUTIC USE OF PROTOZOANS OF THE WILLAERTIA GENUS AS A FUNGISTATIC AND/OR FUNGICIDE

(30) Priorité: 10.08.2017 FR 1757644
(43) Date de publication de la demande: 17.06.2020
(60) Demande divisionnaire: 26198092.4 / 4 813 211
(73) Titulaire: Amoeba, 69680 Chassieu (FR)
(72) Inventeur: PLASSON, Fabrice, 69003 Lyon (FR); MAMERI, Mouh Oulhadj, 69120 Vaulx En Velin (FR)
(74) Mandataire: Plasseraud IP
(86) Numéro de dépôt international: PCT/FR2018/052040
(87) Numéro de publication internationale: WO 2019/030459

(56) Documents cités:
- WO-A1-2015/059411
- WO-A2-2008/043969
- US-A1- 2011 300 106
- J. N. STEENBERGEN ET AL: "Interaction of Blastomyces dermatitidis, Sporothrix schenckii, and Histoplasma capsulatum with Acanthamoeba castellanii", INFECTION AND IMMUNITY, vol. 72, no. 6, 1 June 2004 (2004-06-01), pages 3478 - 3488, XP055441134, ISSN: 0019-9567, DOI: 10.1128/IAI.72.6.3478-3488.2004
- G. GREUB ET AL: "Microorganisms Resistant to Free-Living Amoebae", CLINICAL MICROBIOLOGY REVIEWS., vol. 17, no. 2, 1 April 2004 (2004-04-01), US, pages 413 - 433, XP055441131, ISSN: 0893-8512, DOI: 10.1128/CMR.17.2.413-433.2004

## Description

### Domaine technique de l'invention

La présente invention concerne l'utilisation de protozoaires du genre *Willaertia,* notamment *Willaertia magna,* comme fongistatique et/ou fongicide.

### Contexte

Les champignons (levures et moisissures) sont responsables de nombreuses maladies affectant à la fois les plantes, les animaux et les humains.

De nombreux fongicides ont ainsi été développés pour éliminer les champignons ou limiter leur développement, et par conséquent traiter et/ou prévenir les maladies induites par les champignons.

En revanche, l'utilisation de ces composés n'est souvent pas optimale que ce soit en termes d'efficacité ou en termes de toxicité pour l'homme ou l'environnement.

En effet, l'utilisation massive, agricole ou non, de pesticides ou d'antifongiques chimiques soulève de nombreux problèmes sanitaires et écologiques. Sur le plan environnemental, on constate une contamination croissante des sols et des nappes phréatiques par les molécules contenues dans les produits phytosanitaires. Ces polluants peuvent être à l'origine de perturbations au sein des écosystèmes contaminés, de par leurs effets toxicologiques potentiels sur les organismes non ciblés. Outre les risques écologiques, les dangers liés à l'utilisation et à l'exposition aux pesticides sont également bien documentés et font l'objet d'une mise en garde de l'Organisation Mondiale de la Santé (OMS). Concernant le traitement des mycoses, il est nécessaire de fournir un composé antimycosique qui soit non toxique pour l'homme. De plus, l'utilisation massive de ces composés a comme conséquence l'apparition de souches résistantes à ces produits. L'évolution des populations conduit à terme à des baisses d'efficacité des produits qui peut aller jusqu'à la perte totale.

La prise de conscience des risques écologiques et sanitaires induits par l'utilisation massive de pesticides chimiques a donc contribué à promouvoir depuis quelques années le développement d'une demande pour des produits antifongiques d'origine naturelle, dont l'impact toxicologique et environnemental est plus faible. Ces produits moins nocifs pour l'homme et son environnement présentent également l'avantage d'une utilisation simplifiée, car ils bénéficient d'une règlementation allégée.

II existe donc un besoin important pour le développement de nouvelles compositions fongicide naturelles, à large spectre qui soient à la fois efficaces, simples à mettre en œuvre, et dont les couts de production soient maitrisés.

II serait également avantageux que de telles compositions puissent agir sur les spores, afin de présenter une efficacité optimale.

### Résumé

Dans ce contexte, les inventeurs ont mis en évidence, de façon totalement inattendue, que les protozoaires du genre *Willaertia,* et notamment les protozoaires de l'espèce amibienne *Willaertia magna,* sous forme vivante et/ou sous forme morte et/ou sous forme de lysat cellulaire, possèdent une activité fongistatique et/ou fongicide, en particulier en agissant sur les spores.

Un premier aspect de la présente invention est l'utilisation non-thérapeutique de protozoaires de l'espèce *Willaertia* pour lutter contre un champignon choisi parmi F. solani, C. globosum, P. chrysogenum, P. viticola, P. infestans, T. viride, T. harzianum, A. fumigatus, A.alternata, A.flavus, P. variotii, A.niger, C. albicans, U. botrytis, E. nigrum, B. cinerea, P. roqueforti, C. cladosporioides, E. chevalieri, A. pullulans et une combinaison de ceux-ci.

Un deuxième aspect de la présente invention est le Protozoaires de l'espèce Willaertia magna pour son utilisation comme médicament antimycosique pour le traitement des infections provoquées par les champignons choisis parmi A. fumigatus, A. flavus, C. albicans, U. botrytis, A.niger, P.variotii, A.alternata, et une combinaison de ceux-ci.

Selon la présente divulgation, les protozoaires du genre *Willaertia,* notamment de l'espèce *Willaertia magna,* sous forme vivante et/ou morte et/ou sous forme de lysat cellulaire, peuvent être utilisés de manière préventive ou curative.

Les protozoaires de l'espèce *Willaertia magna,* sous forme vivante ou morte, peuvent être utilisés seuls, ou de façon avantageuse, en combinaison avec d'autres protozoaires, et notamment des protozoaires du genre amibien *Solumitris,* des protozoaires Cercozoa du genre *Cercomonas* ou des protozoaires Cercozoa du genre *Paracercomonas.*

De préférence, les protozoaires utilisés dans le cadre de la présente divulgation correspondent à la souche de *Willaertia magna* déposée le 21 août 2006 sous le numéro PTA-7824 à l'ATCC ou à la souche de *Willaertia magna* déposée le 21 août 2006 sous le numéro PTA-7825 à l'ATCC, ces deux souches ayant été initialement déposées aux noms du CENTRE NATIONAL DE LA RECHERCHE SCIENTIFIQUE (CNRS) - 3 rue Michel Ange - 75794 PARIS CEDEX 16 / France et UNIVERSITE LYON 1 CLAUDE BERNARD - 43 Boulevard du 11 Novembre 1918 - 69622 VILLEURBANNE Cedex / France. Lesdites souches déposées sont également décrites dans la publication de la demande internationale PCT WO2008/043969.

Tel qu'utilisé dans le cadre de la présente divulgation, les termes « végétal » et « végétaux » désignent autant les plantes entières que les parties isolées de ces plantes comme les feuilles, les fruits, les fleurs, les graines, les semences, le tronc, les racines, les tiges.

Tel qu'utilisé dans le cadre de la présente divulgation, le terme « champignon » se réfère aux champignons unicellulaires ou pluricellulaires, incluant les levures et les moisissures, sous forme de spores ou sous forme de mycélium.

Tel qu'utilisé dans le cadre de la présente divulgation, le terme « produit phytopharmaceutique » désigne une préparation ou un mélange de substances destiné :
- à protéger les végétaux contre un ou des organismes nuisibles,
- et / ou à prévenir l'action des organismes nuisibles aux végétaux,
- et / ou à exercer une action sur les processus vitaux des végétaux (dans la mesure où il ne s'agit pas de substances nutritives),
- et / ou à assurer la conservation des produits végétaux.

Tel qu'utilisé dans le cadre de la présente divulgation, le terme « forme vivante » désigne la forme végétative métaboliquement active du protozoaire.

Tel qu'utilisé dans le cadre de la présente divulgation, le terme « forme morte » désigne une forme métaboliquement inactive du protozoaire, qui a été inactivé par un procédé mécanique, physique, thermique ou chimique, par exemple choc osmotique, choc thermique, par ultrasons, ou encore sous contrainte mécanique de type centrifugation par exemple. La « forme morte » peut contenir des cellules mortes entières, du microorganisme, ou du lysat cellulaire, ou un mélange de cellules mortes et entières et de lysat cellulaire. Un lysat désigne communément un matériau obtenu à l'issue de la destruction ou dissolution de cellules biologiques par un phénomène dit lyse cellulaire provoquant ainsi la libération des constituants biologiques intracellulaires naturellement contenus dans les cellules du microorganisme considéré. Au sens de la présente invention, le terme « forme morte » est utilisé indifféremment pour désigner les cellules de *Willaertia* mortes, l'intégralité du lysat obtenu par lyse de *Willaertia* ou seulement une fraction de celui-ci.

La différenciation entre cellules vivantes, cellules mortes et lysat peut notamment se faire par observation microscopique avec une coloration par un marqueur de non-viabilité comme le bleu Trypan (CAS : 72-57-1) qui marque les cellules mortes.

L'utilisation non-thérapeutique trouve, en particulier, de multiples applications :
- pour lutter contre la prolifération des champignons parasites des végétaux, de préférence pour le traitement des maladies fongiques de la vigne, des céréales, des pommes de terre, des arbres fruitiers et des cultures maraichères, de manière encore plus préférentielle pour le traitement des maladies fongiques choisies parmi l'oïdium de la vigne, le mildiou de la vigne, la septoriose et la fusariose des céréales, l'helminthosporiose des céréales, le mildiou et l'alternariose de la pomme de terre, la tavelure du pommier, le Sclerotinia des colza et des cultures légumière et une combinaison de celles-ci.
- pour la désinfection des réseaux (autres que les tours aéro-réfrigérantes) de centrales de traitement de l'air ;
- pour lutter contre les contaminations fongiques et/ou la formation de biofilms fongiques dans les denrées alimentaires, les équipements de transformation/production des denrées alimentaires, les usines de transformation/production des denrées alimentaires, les surfaces venant en contact avec les denrées alimentaires ;
- pour lutter contre les contaminations fongiques et/ou la formation de biofilms fongiques dans les produits cosmétiques, les équipements de transformation/production des produits cosmétiques, les usines de transformation/production des produits cosmétiques, les surfaces venant en contact avec les produits cosmétiques ;

L'utilisation comme médicament antimycosique trouve en particulier application dans le traitement des infections provoquées par les champignons choisis parmi *Aspergillus, Candida, U. botrytis, A.niger, P.variotii, A.alternata,* et une combinaison de ceux-ci, de préférence *Candida,* de manière encore plus préférentielle *Candida albicans.*

L'utilisation trouve aussi application dans le traitement des maladies nosocomiales provoquées par les champignons, en particulier des pathologies respiratoires.

De préférence, les protozoaires du genre *Willaertia,* notamment de l'espèce *Willaertia magna,* sous forme vivante ou morte (notamment sous forme de lysat cellulaire), ou un mélange de formes vivantes et mortes, sont utilisés pour lutter contre un champignon choisi parmi la division des *Ascomycètes,* plus préférentiellement parmi la classe des *sordariomycetes, eurotiomycètes, dothideomycetes, saccharomycetes, leotiomycetes, oomycetes* et une combinaison de celles-ci, encore plus préférentiellement parmi le genre des *Botrytis, Erysiphe, Fusarium, Penicillium, Plasmopara, Phytophthora, Chaetomium, Trichoderma, Aspergillus, Alternaria, Candida, Ulocladium, Epicoccum, Cladosporium* et une combinaison de ceux-ci et de manière encore plus préférée parmi *B. cinerea, E. necator, F. solani, C. globosum, P. chrysogenum, P. expansum, P. viticola, P. infestans, T. viride, T. harzianum, A. fumigatus, A.alternata, Aflavus, P. variotii, A.niger, C. albicans, U. botrytis, E. nigrum, B. cinerea, P. roqueforti, C. cladosporioides, E. chevalieri, A. pullulans* et une combinaison de ceux-ci.

Dans un mode de réalisation particulier, les protozoaires de l'espèce *Willaertia magna* sont utilisés soit sous forme vivante, soit sous forme morte (notamment sous forme de lysat cellulaire), soit en mélange de formes vivantes et mortes. Dans un mode de réalisation particulier, le mélange utilisé comprend 50% ou plus de protozoaires sous forme morte, notamment sous forme de lysat cellulaire, par exemple 60% ou plus, 70% ou plus, 80% ou plus, 90% ou plus, 95% ou plus de protozoaires sous forme morte (par rapport au nombre de protozoaires totaux avant inactivation), notamment sous forme de lysat cellulaire.

Dans un mode de réalisation particulier, les protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre les maladies fongiques affectant les végétaux. De préférence les végétaux sont choisis parmi les graminées, les dicotylédones, les plantes annuelles, bisannuelles et pérennes, les plants de légumes ou les légumes récoltés, les plantes ou arbres à fruits ou les fruits récoltés, les céréales, les oléagineux, les protéagineux, les plantes ligneuses, et sont notamment choisis parmi les plants ou produits issus de pomme de terre, betterave, canne à sucre, tabac, vigne, blé, colza, orge, riz, maïs, sorgho, millet, soja, haricot, tomate, concombre, laitue, fraisier, pommier, poirier, agrumes, banane, ananas, pêcher, abricotier, cerisier, noyer, et noisetier.

Dans un mode de réalisation particulier, les protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre les champignons affectant les surfaces, les matériaux, les équipements et/ou les systèmes de ventilation.

Dans un mode de réalisation particulier, les protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre les champignons affectant la santé humaine ou animale.

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *F*. *solani* pour le traitement des maladies fongiques des végétaux (céréales et maïs).

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *C*. *globosum* pour le traitement des maladies fongiques des végétaux, de préférence affectant les céréales ou le riz ou pour le traitement des contaminations des produits à base de cellulose tels que le papier et les matériaux de construction.

Dans un autre mode de réalisation particulier, les protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *P. viticola* pour le traitement des maladies fongiques des végétaux, de préférence affectant la vigne.

Dans un autre mode de réalisation particulier, les protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *P. infestans* pour le traitement des maladies fongiques des végétaux, de préférence affectant la pomme de terre.

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre les moisissures du genre *Penicillium* comme *P. chrysogenum,* du genre *Aspergillus* comme *Aspergillus niger,* ou du genre *Cladosporium* comme *Cladosporium cladosporioides* pour le traitement des contaminations des environnements intérieurs tels que les murs, les matériaux, les équipements et les systèmes de ventilation, etc.

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *T*. *viride* et/ou *T. harzianum* pour le traitement des contaminations des denrées alimentaires et des environnements intérieurs tels que les murs, les matériaux, les équipements et les systèmes de ventilation , etc.

Dans un autre mode de réalisation particulier, les protozoaires du genre *Willaertia,* notamment de l'espèce *Willaertia magna* sous forme vivante ou sous forme morte ou sous forme de lysat cellulaire, ou en mélange de formes vivantes, et / ou de formes mortes et / ou de lysat cellulaire, sont utilisés pour lutter contre *A*. *fumigatus* pour le traitement des maladies nosocomiales, en particulier des pathologies respiratoires.

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *A.alternata* pour le traitement des maladies fongiques des végétaux, de préférence l'alternariose de la pomme de terre ou le traitement des lésions cutanées, mycoses et infections de l'appareil respiratoire supérieur chez l'homme.

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *P*. *variotii* pour le traitement des contaminations fongiques et/ou la formation de biofilms fongiques dans les denrées alimentaires, de préférence les céréales ou pour le traitement des mycoses, pneumonies, sinusites chez l'homme.

Dans un autre mode de réalisation particulier, les protozoaires du genre *Willaertia,* notamment de l'espèce *Willaertia magna,* sous forme vivante ou sous forme morte ou sous forme de lysat cellulaire, ou en mélange de formes vivantes, et / ou de formes mortes et / ou de lysat cellulaire, sont utilisés pour lutter contre *A.niger* pour le traitement des contaminations fongiques et/ou la formation de biofilms fongiques dans les denrées alimentaires, des environnements intérieurs tels que les murs, les matériaux, les équipements et les systèmes de ventilation, etc. ou pour le traitement des mycoses de l'oreille interne ou aspergillose pulmonaires chez l'homme.

Dans un autre mode de réalisation particulier, les protozoaires du genre *Willaertia,* notamment de l'espèce *Willaertia magna,* sous forme vivante ou morte, sont utilisés pour lutter contre *C*. *albicans* pour le traitement des mycoses chez l'homme.

Dans un autre mode de réalisation particulier, les protozoaires du genre *Willaertia,* notamment de l'espèce *Willaertia magna,* sous forme vivante ou morte, sont utilisés pour lutter contre *U. botrytis* pour le traitement des maladies fongiques des végétaux, le traitement des contaminations fongiques et/ou la formation de biofilms fongiques dans les denrées alimentaires et pour le traitement de l'onychomycose.

Dans un autre mode de réalisation particulier, les protozoaires du genre *Willaertia,* notamment de l'espèce *Willaertia magna,* sous forme vivante ou morte, sont utilisés pour lutter contre *E. nigrum ou A. pullulans* pour le traitement des contaminations fongiques et/ou la formation de biofilms fongiques dans les denrées alimentaires ou les cosmétiques ou de la contamination des environnements intérieurs tels que les murs, les matériaux, les équipements et les systèmes de ventilation, etc.

Dans un autre mode de réalisation particulier, les protozoaires du genre *Willaertia,* notamment de l'espèce *Willaertia magna,* sous forme vivante ou morte, sont utilisés pour lutter contre *A.flavus, P. roqueforti, B. cinerea, C. cladosporioides ou E. chevalieri* pour le traitement des contaminations fongiques et/ou la formation de biofilms fongiques dans les denrées alimentaires ou les cosmétiques.

La présente divulgation vise également une composition phytopharmaceutique , qui ne fait pas partie de la présente invention, comprenant une quantité efficace de protozoaires de l'espèce *Willaertia magna,* sous forme vivante ou sous forme morte ou sous forme de lysat cellulaire, ou en mélange de formes vivantes, et / ou de formes mortes et / ou de lysat cellulaire.

Dans un mode de réalisation particulier, la composition phytopharmaceutique contient une quantité efficace de protozoaires de l'espèce *Willaertia magna,* sous forme de lysat cellulaire.

Dans un mode de réalisation particulier, la composition phytopharmaceutique comprend également un ou plusieurs agents de formulation, notamment de l'eau, et, le cas échéant, un ou plusieurs adjuvants actifs supplémentaires.

Dans un mode de réalisation particulier, la composition phytopharmaceutique est une composition à formulation sèche solide, de préférence sous forme déshydratée ou lyophilisée. La composition phytopharmaceutique sous forme sèche est destinée à être reconstitué avec un solvant de reconstitution avant utilisation pour être prêt à l'emploi. Le solvant de reconstitution est de préférence de l'eau.

Dans un autre mode de réalisation particulier, la composition phytopharmaceutique est une composition à formulation liquide concentrée. La composition phytopharmaceutique sous forme liquide concentré est destinée à être dilué avec un solvant de dilution avant utilisation pour être prêt à l'emploi. Le solvant de dilution est de préférence de l'eau.

Dans un autre mode de réalisation particulier, la composition phytopharmaceutique est une composition à formulation liquide directement prête à l'emploi.

La composition phytopharmaceutique décrit ci-dessus, sous sa forme finale prête à l'emploi, peut être appliquée aux végétaux de différentes manières et selon différents programmes de traitements. En particulier, l'application de la composition phytopharmaceutique peut se faire avant ou après récolte sur les feuilles, le sol, les fleurs, les branches, les tiges, le tronc, les racines et/ou les fruits. L'application de la composition phytopharmaceutique se fait de préférence par pulvérisation. L'application de la composition phytopharmaceutique peut également se faire sous forme de mélange à d'autres produits phytopharmaceutiques, à des engrais, à l'eau d'arrosage, ou autre.

Pour les utilisations non-thérapeutiques, les protozoaires de l'espèce *Willaertia magna,* sous forme vivante ou morte, pourront être directement mis en contact sur les équipements de transformation/production et les surfaces venant en contact avec les denrées alimentaires ou les cosmétiques, ou sur les végétaux à traiter. Le traitement peut être fait par exemple par pulvérisation, par exemple sous la forme d'une solution aqueuse en aérosol. De tels protozoaires pourront donc être également utilisés en combinaison avec d'autres agents désinfectants et/ou pesticide.

La présente divulgation vise également une composition pharmaceutique , qui ne fait pas partie de la présente invention, comprenant une quantité efficace des protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, comme antimycosiques et un véhicule pharmaceutiquement acceptable. Ladite composition pharmaceutique peut être en particulier un pansement à substance active, un cataplasme, un emplâtre médicamenteux, un gel, une pâte, une lotion, une mousse, une huile, une émulsion, solution aqueuse, suspension aqueuse, crème, pommade, poudre ou spray à appliquer sur la peau ou les muqueuses.

Pour l'utilisation comme médicament antimycosique, les protozoaires, notamment de l'espèce *Willaertia magna,* sous forme vivante ou sous forme morte ou sous forme de lysat cellulaire, ou en mélange de formes vivantes, et / ou de formes mortes et / ou de lysat cellulaire, pourront être appliqués sur les lésions de manière topique sur la peau ou les muqueuses (par exemple sous la forme d'une crème, pommade, spray ou poudre à appliquer sur la peau ou les muqueuses, typiquement les muqueuses vaginales ou anales).

La divulgation vise également une composition pharmaceutique , qui ne fait pas partie de la présente invention, comprenant une quantité efficace des protozoaires comme antimycosiques et un véhicule pharmaceutiquement acceptable. Ladite composition pharmaceutique peut être en particulier un pansement à substance active, un cataplasme, un emplâtre médicamenteux, un gel, une pâte, une lotion, une mousse, une huile, une émulsion, solution aqueuse, suspension aqueuse, crème, pommade, poudre ou spray à appliquer sur la peau ou les muqueuses.

Un autre objet de la présente divulgation est un procédé de lutte contre la prolifération des champignons, à l'exception des méthodes de traitement appliquées au corps humain ou animal, comprenant une étape de mise en contact de protozoaires de l'espèce *Willaertia magna,* sous forme vivante ou sous forme morte ou sous forme de lysat cellulaire, ou en mélange de formes vivantes, et / ou de formes mortes et / ou de lysat cellulaire, avec lesdits champignons.

### Description des figures

**Figure 1** : Effet des amibes sur la germination de *B. cinerea.* Tests en microplaque en présence des spores de *B. cinerea* sans amibes. Observation après 24H d'incubation. **Souches sensibles** : B05.10 et M421. **Souche résistante** : M426. (fongicide inhibiteur de la respiration)
**Figure 2** : Effet des amibes sur la germination de *B. cinerea.* Tests en microplaque en présence des spores de *B. cinerea +* 500 amibes dans milieu MC et spores de *B. cinerea +.* **Souches sensibles** : B05.10 et M421. **Souche résistante** : M426. (fongicide inhibiteur de la respiration). 50000 amibes dans milieu MC. Observation après 24H d'incubation.
**Figure 3** : Effet du milieu de culture (MC) sur la germination de *B. cinerea souches sensibles.* Tests en microplaque en présence des spores de *B. cinerea* + milieu de culture (MC). **Souches sensibles** : B05.10 et M421. Observation après 24H d'incubation.

### Exemples

Les exemples ci-après permettent d'illustrer l'invention .

### Exemple 1 : Analyse qPCR de l'ADN fongique des interactions champignon-Willaertia sur 19 souches.

Les essais d'interaction sur 19 souches de champignons (tableau 1) ont été réalisés en mettant les spores en contact avec l'amibe *«Willaertia magna»* référence ATCC PTA-7824 selon un ratio amibe/spore = 10 amibes / spores, en plaques de 24 puits.

Dans une première plaque, des spores seules de chaque souche ont été incubées dans les mêmes conditions (dans 2 ml de MEAc). Après 3H de contact, la solution est centrifugée à 14 000 rpm pendant 5 minutes. Le surnageant est ensuite éliminé et le culot repris dans 200µl pour l'extraction d'ADN.

Pour les essais à 24H réalisés dans une seconde plaque, le surnageant est éliminé directement du puits après 24 heures et les « mélanges spores-amibes » sont repris dans 200µl pour extraire l'ADN. Un suivi par observations microscopiques est effectué à chaque étape. Une extraction d'ADN est effectuée sur les échantillons (200µl) à l'aide du kit « NucleoSpin plant II - Macherey-Nagel» selon les instructions du fabricant après une étape de broyage avec du sable fin.

L'amplification par qPCR de l'ADN des champignons seuls et des complexes *Willaertia-champignons* a été effectuée sur un appareil CFX96-Biorad. La quantification est réalisée dans un volume final de 20µl contenant 5µl d'ADN, les amorces (300nM) et 10µl de SybrGreen-Mix. Le programme suivant a été utilisé : une étape de dénaturation 95°C pendant 10 minutes avec une étape d'amplification « 95°C pendant 15 secondes + 60°C pendant 1 minute » de 35 cycles.

Les tests effectués sur les 19 souches de champignons sont réalisés selon le protocole validé dans l'étape précédente. Les plaques sont observées au microscope avant de lancer l'analyse moléculaire. Les résultats sont des courbes qPCR obtenues en triplicat pour chaque condition (spores-3H, spores-24H, spore-amibe3H et spore-amibe24H). Étant donné que l'analyse qPCR utilise des amorces universelles, une gamme du champignon P.chrysogenum a été utilisée pour estimer approximativement la quantité d'ADN amplifié pour chaque essai. Ces données permettent d'évaluer le passage du stade spore au mycélium dans les témoins sans amibe ainsi que le degré d'inhibition de la germination en présence des amibes.

**Résultats et conclusion** : Tableau 1: Ecart d'amplification qPCR entre les essais réalisés sur les spores seules (écart entre 3 et 24 heures) ou en interaction avec les amibes après 24H de contact. ΔCq (3H-24H) : écart en nombre de cycles entre les spores à 3H et 24H. ΔCq (T24H- *Willaertia*24H) : écart en nombre de cycles entre les spores seules (T24H) et en présence d'amibes (Amibes 24H) après 24H d'incubation.

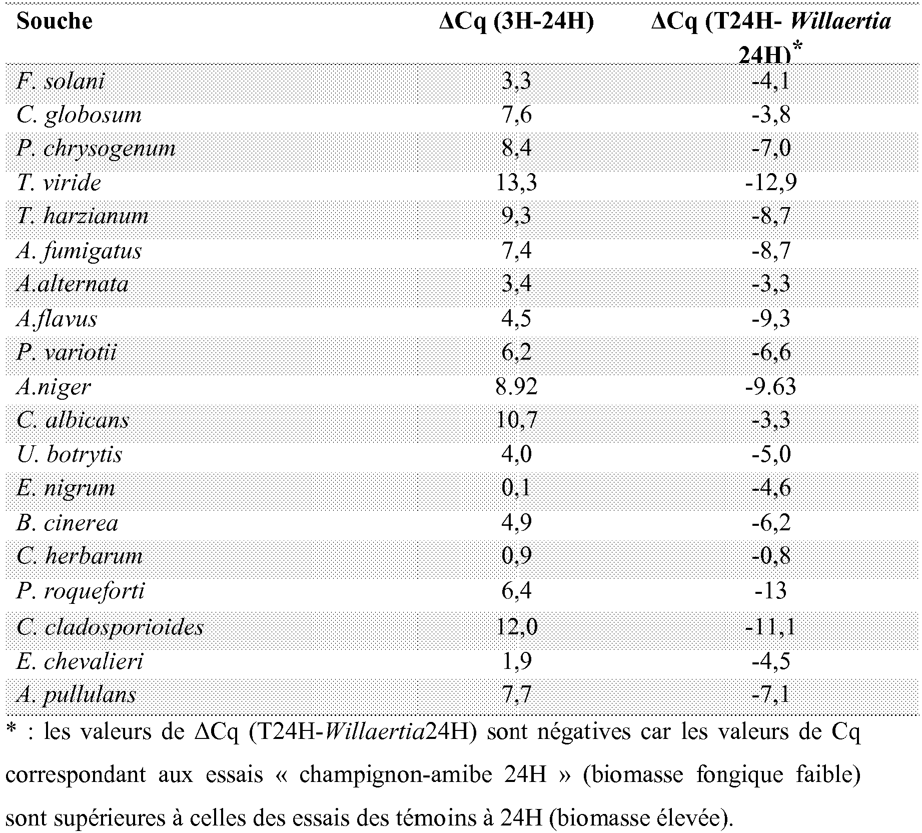

L'analyse par qPCR a permis de quantifier la biomasse fongique et de différencier les stades de développement des champignons et de montrer l'effet fongistatique et/ou fongicide sur 19 souches traitées avec des écarts très important. L'écart en nombre de cycles Cq entre les deux essais sans et avec amibes est très important et dans certains cas dépasse les 10 cycles. Ces résultats reflètent la présence d'une biomasse fongique très faible en présence des amibes après 24H d'incubation suite à l'inhibition de la germination des spores. La différence entre le stade spores à 3H et le mycélium développé après 24H est nettement constatée par la différence d'amplification entre les spores à 3H et 24H sur des souches testées. Les résultats de qPCR reflètent parfaitement les observations visuelles réalisées au microscope pour mettre au point ce protocole.

### Exemple 2 : Etude de l'effet de Willaertia magna sur la croissance de Botrytis cinerea.

**Principe** : Les essais réalisés sont inspirés des tests utilisés au laboratoire pour évaluer l'efficacité des molécules fongicides conventionnelles. L'effet de l'amibe *Willaertia magna* (souche ATCC PTA-7824) sur la germination des spores de *B. cinerea* est analysé en mettant en contact différentes concentrations d'amibes avec des spores du champignon. Après 24 heures d'incubation, la germination des spores de *B. cinerea* en présence d'amibe est comparée au témoin sans amibes.

**Protocole** : Les souches de *B. cinerea* sont maintenues sur milieu de culture (à base de flocons d'avoine) sous une alternance lumière du jour-UV (12H/12H) à 21°C. Les souches, de phénotypes sensible ou résistant à un fongicide appartenant aux inhibiteurs de la respiration cellulaire. Les références des souches testées sont : souche B05.10 (sensible), M421 (sensible isolée du champ) et M426 (résistante isolée du champ).

Pour tester l'effet de l'amibe *Willaertia magna* sur la germination des conidies de *B. cinerea,* les spores du champignon sont incubées avec différentes concentrations d'amibes. Dans une plaque de 96 puits, 500 spores de *B. cinerea* sont mises en contact avec 5^{E}+02 et 5^{E}+04 amibes par puits dans un volume final de 50µl. Les amibes testées sont préparées dans du milieu de culture (MC).

Les témoins suivants sont également préparés dans les mêmes conditions :
- Spores de *B. cinerea* dans un milieu de culture liquide YBA
- Spores de *B. cinerea* dans milieu de culture (MC)
- Spores de *B. cinerea* avec le fongicide inhibiteur de la respiration

Chaque condition est testée en triplicat. Les plaques sont incubées à obscurité à 21°C pendant 24H ou 48H. La germination des conidies de *B. cinerea* est observée sous microscope inversé et une série de photos est effectuée pour chaque condition.

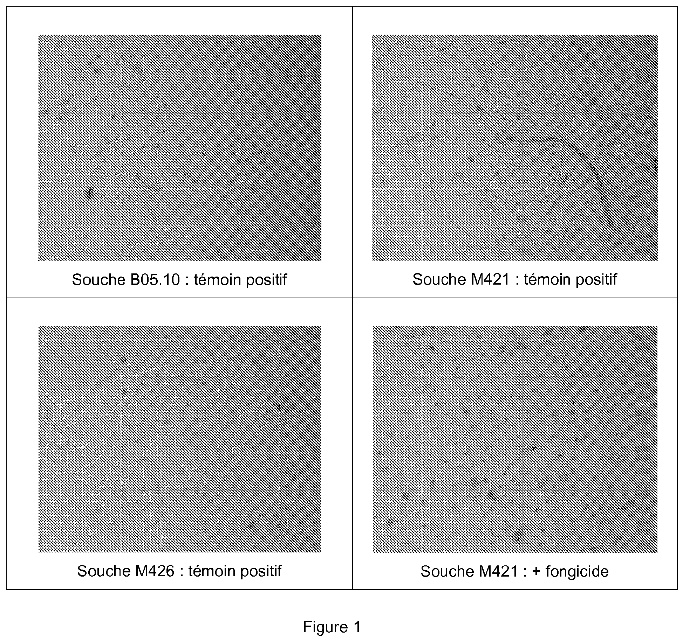

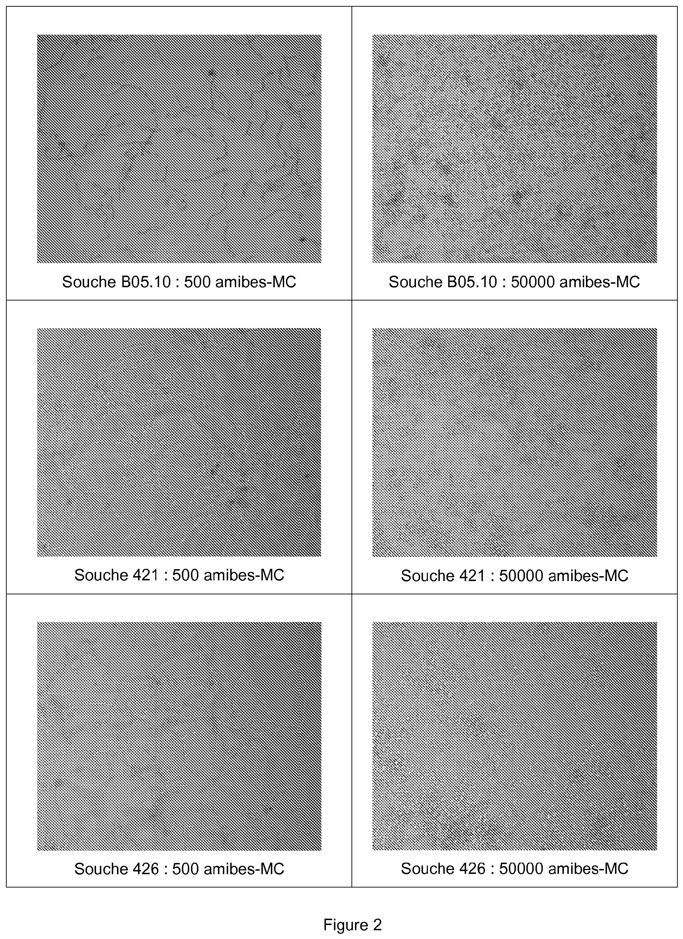

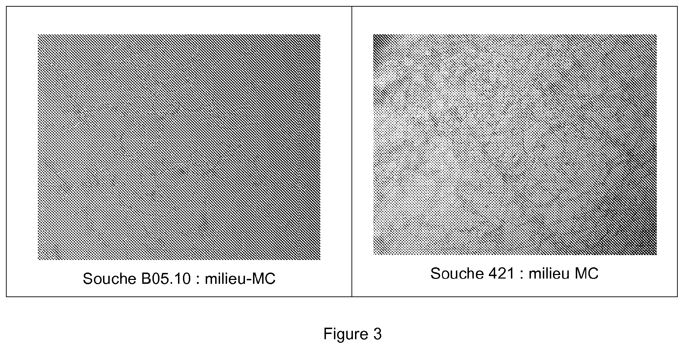

**Résultats et conclusion** : L'observation des spores après 24H a permis de mettre en évidence un ralentissement de la germination du champignon à fortes concentration d'amibes. Quelle que soit la souche de *B. cinerea,* cet effet est beaucoup plus visible quand le champignon est en contact avec des quantités d'amibes formant un tapis confluent (figure 2). Avec des rapports plus faibles (500 amibes) l'effet est moins visible comparé aux témoins (figure 1 et figure 2). La souche B05.10 est une souche de collection et elle est moins vigoureuse/vivace. Comparée aux souches sauvages (prélevées au champ), elle mettra plus de temps pour se développer. Le milieu de culture (MC) seul n'affecte pas la croissance du champignon, le milieu de culture semble même stimuler la germination des spores de *B. cinerea* (figure 3).

Ces résultats montrent une efficacité de *Willaertia magna* sur la germination de *B. cinerea* dans les conditions expérimentales, avec un effet dose qualitatif observé.

### Exemple 3 : Efficacité de Willaertia magna sur le mildiou de la vigne (P. viticola) :

**Principe** : Les essais sont effectués sur de jeunes plants de Cabernet sauvignon. Chaque groupe de plantes est traité dans des conditions contrôlées avec un pulvérisateur à manche. Après traitement des plants par les solutions testées, une infection volontaire des plantes par du mildiou de la vigne est réalisée. Après 7 à 10 jours d'incubation, la gravité de la maladie est évaluée pour évaluer l'efficacité de différents produits par rapport à la gravité de la maladie sur les plantes non traitées.

### Protocole :

Les boutures de plants de Cabernet sauvignon sont produites en serre (température 15°C - 30°C) et 6 groupes de 6 plantes sont constitués pour l'étude de chaque condition d'essai.

Les cellules de *Willaertia magna* sont cultivées dans un milieu de culture jusqu'à obtenir la concentration désirée de 9E+09 cellules par litre.

Divers échantillons sont testés :
1. Contrôle négatif, condition 1 : sans traitement.
2. Contrôle du milieu, condition 2 : milieu de dilution
3. Fongicide, condition 3 : *Willaertia magna* dans du milieu de culture
4. Fongicide, condition 4 : échantillon de la condition 3, dilué dans du milieu de dilution
*5.* Fongicide, condition 5 : un échantillon de la condition 3 est mis sous contrainte mécanique pour tuer les cellules de *Willaertia.* L'échantillon est passé sous pression dans une vanne à vide poussé, quasiment fermée, à l'aide d'une pompe, entrainant la mort d'une partie des cellules et la lyse d'une partie des cellules mortes. L'échantillon obtenu est observé au microscope, avec un marqueur de non-viabilité (bleu Trypan) pour comptage des cellules vivantes restantes (non marquées au Bleu Trypan)
6. Fongicide, condition 6 : un échantillon de la condition 4 est mis sous contrainte mécanique pour tuer les cellules de *Willaertia.* L'échantillon est passé sous pression dans une vanne à vide poussé, quasiment fermée, à l'aide d'une pompe, entrainant la mort d'une partie des cellules et la lyse d'une partie des cellules mortes. L'échantillon obtenu est observé au microscope, avec un marqueur de non-viabilité (bleu Trypan) pour comptage des cellules vivantes restantes (non marquées au Bleu Trypan)
7. Contrôle positif, condition 6 : bouillie bordelaise équivalent à 3 kg/ha.

Chaque échantillon est appliqué sur un des groupes de 6 plantes par pulvérisation sur la plante entière.

24 heures après, une suspension de sporanges de P. viticola est pulvérisé sur la face inférieure des feuilles. Les plants sont ensuite placés en incubation à 24 ± 5°C, éclairés 14 heures par jours, dans des chambres individuelles à une humidité favorable au développement de la maladie. Après 7 à 10 jours, une évaluation de l'efficacité fongicide dans chaque condition est faite, pour chaque plante et chaque stade de feuille. L'évaluation est basée sur des observations visuelles de la lésion fongique, constituée de deux éléments : la surface infectée et l'intensité de la sporulation. Une échelle de 0 à 100% (par pas de 5%) est utilisée ; chaque feuille étant observée individuellement. L'efficacité est calculée selon la formule suivante : Efficacité = 100 x [(NT-T) / NT] (T étant le taux d'attaque moyen sur les plants non traités et T le taux moyen d'attaque sur les plants traités). Les résultats sont présentés dans le tableau 2.

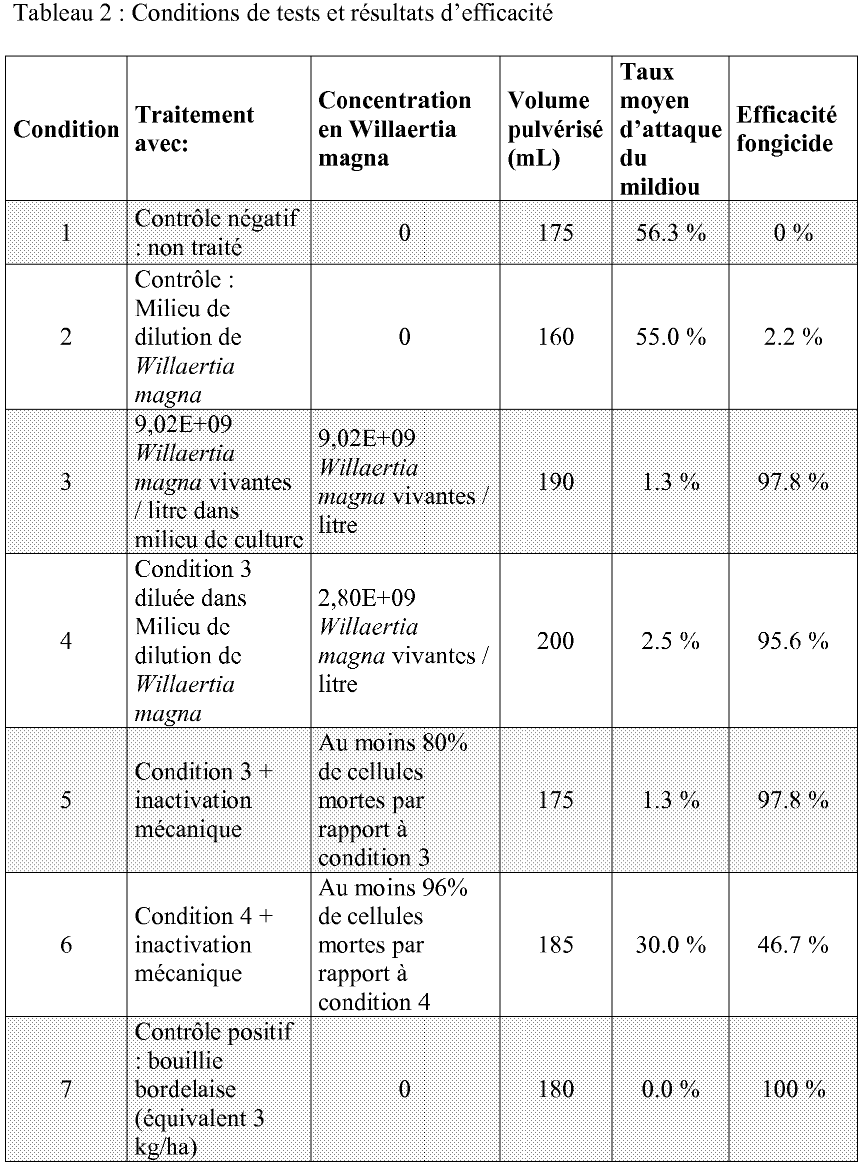

**Résultats et conclusion** : La sévérité de la maladie est élevée (56,3%) sur les plantes non traitées. Cette valeur est également élevée (55%) après pulvérisation du milieu de dilution.

Une très faible quantité de maladie est observée sur les plantes traitées avec *Willaertia magna,* sous forme vivante : l'infection par P. viticola est de 2,5 à 1,3% de la surface des feuilles (versus 56% pour le contrôle négatif non traité). Soit une efficacité de plus de 95%, proche de l'efficacité du fongicide de référence (Bouillie bordelaise à 3 kg / ha) qui permet un contrôle parfait du mildiou de la vigne avec une efficacité de 100% (pas d'attaque de mildiou).

Une faible quantité de maladie est observée sur les plantes traitées avec *Willaertia magna* sous forme morte, inactivée par action mécanique par passage dans une pompe et vanne à vide poussé : l'infection par P. viticola est de 30% jusqu'à seulement 1,3% de la surface des feuilles (versus 56% pour le contrôle négatif non traité).

En conclusion, *Willaertia magna,* sous forme vivante et sous forme morte, fournit une activité élevée contre le mildiou de la vigne dans les conditions expérimentales.

## Revendications

1. Utilisation non-thérapeutique de protozoaires de l'espèce *Willaertia magna,* comme fongistatique et/ou fongicide pour lutter contre un champignon choisi parmi *F. solani, C. globosum, P. chrysogenum, P. viticola, P. infestans, T. viride, T. harzianum, A. fumigatus, A.alternata, A.flavus, P. variotii, A.niger, C. albicans, U. botrytis, E. nigrum, B. cinerea, P. roqueforti, C. cladosporioides, E. chevalieri, A. pullulans* et une combinaison de ceux-ci.

2. Utilisation selon la revendication 1, **caractérisée en ce que** des protozoaires de l'espèce *Willaertia magna* sont utilisés soit sous forme vivante, soit sous forme morte, soit sous forme de lysat cellulaire, soit en mélange de formes vivantes et / ou de formes mortes et / ou de lysat cellulaire.

3. Utilisation selon la revendication 1 ou 2, **caractérisée en ce que** des protozoaires de l'espèce *Willaertia magna* sont utilisés en association avec d'autres protozoaires, par exemple des protozoaires du genre amibien *Solumitris,* des protozoaires cercozoaires du genre *Cercomonas* ou des protozoaires cercozoaires du genre *Paracercomonas.*

4. Utilisation selon l'une des revendications 1 à 3, **caractérisée en ce que** ces protozoaires correspondent, à la souche déposée sous le numéro PTA-7824 à l'ATCC ou à la souche déposée sous le numéro PTA-7825 à l'ATCC.

5. Utilisation selon l'une des revendications 1 à 4, pour lutter contre *B*. *cinerea, F. solani, C. globosum, P. viticola, P. infestans, A.alternata, A.flavus* ou *U. botrytis* pour le traitement des maladies fongiques des végétaux.

6. Utilisation selon l'une des revendications 1 à 4,
(i) pour la désinfection des réseaux de centrales de traitement de l'air,
(ii) pour lutter contre les contaminations fongiques et/ou la formation de biofilms fongiques dans les denrées alimentaires, les équipements de transformation/production des denrées alimentaires, les usines de transformation/production des denrées alimentaires ou des produits cosmétiques, les surfaces venant en contact avec les denrées alimentaires ou les produits cosmétiques, ou,
(iii) pour le traitement des contaminations des environnements intérieurs.

7. Protozoaires de l'espèce *Willaertia magna* pour son utilisation comme médicament antimycosique pour le traitement des infections provoquées par les champignons choisis parmi *A*. *fumigatus, A. flavus, C. albicans, U. botrytis, A.niger, P.variotii, A.alternata,* et une combinaison de ceux-ci.

## Patentansprüche

1. Nichttherapeutische Verwendung von Protozoen der Spezies *Willaertia magna* als fungistatisch und/oder fungizid zur Bekämpfung eines Pilzes, ausgewählt aus *F. solani*, *C. globosum*, *P. chrysogenum*, *P. viticola*, *P. infestans*, *T. viride*, *T. harzianum*, *A. fumigatus*, *A.alternata*, *A.flavus*, *P. variotii*, *A.niger*, *C. albicans*, *U. botrytis*, *E. nigrum*, *B. cinerea*, *P. roqueforti*, *C. cladosporioides*, *E. chevalieri*, *A. pullulans* und einer Kombination davon.

2. Verwendung nach Anspruch 1, **dadurch gekennzeichnet, dass** Protozoen der Spezies *Willaertia magna* entweder in lebender Form, oder in toter Form, oder in Form eines Zelllysats, oder in einer Mischung aus lebenden Formen und/oder toten Formen und/oder Zelllysat verwendet werden.

3. Verwendung nach Anspruch 1 oder 2, **dadurch gekennzeichnet, dass** Protozoen der Spezies *Willaertia magna* in Verbindung mit anderen Protozoen verwendet werden, beispielsweise mit amöboiden Protozoen der Gattung *Solumitris*, cercozoischen Protozoen der Gattung *Cercomonas* oder cercozoischen Protozoen der Gattung *Paracercomonas*.

4. Verwendung nach einem der Ansprüche 1 bis 3, **dadurch gekennzeichnet, dass** diese Protozoen dem bei der ATCC unter der Nummer PTA-7824 hinterlegten Stamm oder dem bei der ATCC unter der Nummer PTA-7825 hinterlegten Stamm entsprechen.

5. Verwendung nach einem der Ansprüche 1 bis 4 zur Bekämpfung von *B. cinerea*, *F. solani*, *C. globosum*, *P. viticola*, *P. infestans*, *A.alternata*, *A.flavus* oder *U. botrytis* zur Behandlung von Pilzkrankheiten von Pflanzen.

6. Verwendung nach einem der Ansprüche 1 bis 4,
(i) zur Desinfektion von Netzen von Luftbehandlungsanlagen,
(ii) zur Bekämpfung von Pilzkontaminationen und/oder der Bildung von Pilzbiofilmen in Lebensmitteln, Verarbeitungs-/Produktionsausrüstung für Lebensmittel, Verarbeitungs-/Produktionswerken für Lebensmittel oder kosmetische Produkte, Oberflächen, die mit Lebensmitteln oder kosmetischen Produkten in Kontakt kommen, oder,
(iii) zur Behandlung von Kontaminationen von Innenräumen.

7. Protozoen der Spezies *Willaertia magna* zu ihrer Verwendung als antimykotisches Medikament zur Behandlung von Infektionen, die durch die Pilze, ausgewählt aus *A. fumigatus*, *A. flavus*, *C. albicans*, *U. botrytis*, *A.niger*, *P.variotii*, *A.alternata*, und einer Kombination davon, verursacht werden.

## Claims

1. Non-therapeutic use of protozoa of the species *Willaertia magna*, as fungistatic and/or fungicide to combat a fungus selected from *F. solani*, *C. globosum*, *P. chrysogenum*, *P. viticola*, *P. infestans*, *T. viride*, *T. harzianum*, *A. fumigatus*, *A.alternata*, *A.flavus*, *P. variotii*, *A.niger*, *C. albicans*, *U. botrytis*, *E. nigrum*, *B. cinerea*, *P. roqueforti*, *C. cladosporioides*, *E. chevalieri*, *A. pullulans* and a combination thereof.

2. Use according to claim 1, **characterized in that** protozoa of the species *Willaertia magna* are used either in living form, or in dead form, or in the form of a cell lysate, or in a mixture of living forms and/or dead forms and/or cell lysate.

3. Use according to claim 1 or 2, **characterized in that** protozoa of the species *Willaertia magna* are used in association with other protozoa, for example amoebic protozoa of the genus *Solumitris*, cercozoan protozoa of the genus *Cercomonas* or cercozoan protozoa of the genus *Paracercomonas*.

4. Use according to any one of claims 1 to 3, **characterized in that** these protozoa correspond to the strain deposited under number PTA-7824 with the ATCC or to the strain deposited under number PTA-7825 with the ATCC.

5. Use according to any one of claims 1 to 4, to combat *B. cinerea*, *F. solani*, *C. globosum*, *P. viticola*, *P. infestans*, *A.alternata*, *A.flavus* or *U. botrytis* for the treatment of fungal diseases of plants.

6. Use according to any one of claims 1 to 4,
(i) for the disinfection of air handling unit networks,
(ii) to combat fungal contaminations and/or the formation of fungal biofilms in foodstuffs, foodstuff processing/production equipment, foodstuff processing/production plants or cosmetic products, surfaces coming into contact with foodstuffs or cosmetic products, or,
(iii) for the treatment of indoor environment contaminations.

7. Protozoa of the species *Willaertia magna* for its use as an antimycotic medicament for the treatment of infections caused by the fungi selected from *A. fumigatus*, *A. flavus*, *C. albicans*, *U. botrytis*, *A.niger*, *P.variotii*, *A.alternata*, and a combination thereof.
